(19) Europäisches Patentamt
European Patent Office
Office européen des brevets

(11) EP 1 590 047 B2

(12) NOUVEAU FASCICULE DE BREVET EUROPEEN
Après la procédure d'opposition

(45) Date de publication et mention de la décision concernant l'opposition:
21.08.2013 Bulletin 2013/34

(45) Mention de la délivrance du brevet:
07.04.2010 Bulletin 2010/14

(21) Numéro de dépôt: 03796160.4

(22) Date de dépôt: 04.12.2003

(51) Int Cl.:
A61P 37/02 (2006.01)     A61K 39/40 (2006.01)
C12N 1/20 (2006.01)     C12R 1/23 (2006.01)
C12R 1/25 (2006.01)     A61K 35/74 (2006.01)
A23C 9/20 (2006.01)     A23L 1/30 (2006.01)
C12R 1/01 (2006.01)     C12R 1/225 (2006.01)

(86) Numéro de dépôt international:
PCT/FR2003/003589

(87) Numéro de publication internationale:
WO 2004/052462 (24.06.2004 Gazette 2004/26)

(54) **COMPOSITION COMPRENANT DES LACTOBACILLUS OU DES BIFIDOBACTERIUM ET SON UTILISATION**

ZUSAMMENSETZUNG ENTHALTEND LACTOBACILLUS ODER BIFIDOBACTERIUM UND DEREN VERWENDUNG

COMPOSITION COMPRISING LACTOBACILLI OR BIFIDOBACTERIA AND USE THEREOF

(84) Etats contractants désignés:
AT BE BG CH CY CZ DE DK EE ES FI FR GB GR HU IE IT LI LU MC NL PT RO SE SI SK TR

(30) Priorité: 05.12.2002 FR 0215363
05.12.2002 US 310549

(43) Date de publication de la demande:
02.11.2005 Bulletin 2005/44

(73) Titulaire: DuPont Nutrition Biosciences ApS
1001 Copenhagen K. (DK)

(72) Inventeurs:
• LEYER, Grégory
Madison, WI 53711 (US)
• DENNIN, Véronique
F-59650 Villeneuve d'Ascq (FR)
• NUTTEN, Sophie
F-59114 Steenvoorde (FR)
• POT, Bruno
B-8200 Sint-Michiels-Brugges (BE)
• MERCENIER, Annick
CH-1066 Epalinges (CH)

(74) Mandataire: Williams, Aylsa et al
D Young & Co LLP
Briton House
Briton Street
Southampton SO14 3EB (GB)

(56) Documents cités:
EP-A- 0 856 259        WO-A1-99/17788
US-A- 6 132 710        US-A1- 2001 014 322
US-A1- 2002 006 432    US-B1- 6 399 124

• SHIFFRIN E J ET AL: "Immune modulation of blood leukocytes in humans by lactic acid bacteria: criteria for strain selection" AMERICAN JOURNAL OF CLINICAL NUTRITION, BETHESDA,MD, US, vol. 66, no. 2, août 1997 (1997-08), pages 515S-520S, XP002194826 ISSN: 0002-9165
• PATIDAR SK, PRAJAPATI JB: "Methods for assessing the immunostimulating properties of dietary lactobacili - A critical appraisal" J. FOOD SCI. TECHNOL., vol. 34, no. 3, 1997, pages 181-194, XP009013023
• M.E.SANDERS ET AL: 'The Scientific basis of Lactobacillus acidophilus NCFM Functionality as a Probiotic' DAIRY AND FOOD CULTURE TECHNOLOGIES 2000, pages 319 - 331
• C. DANIEL ET AL: 'Selecting Lactic Acid Bacteria for Their safety and Functionality by Use of a Mouse Colitis Model' APPLIED AND ENVIRONMENTAL MICROBIOLOGY vol. 72, no. 9, Septembre 2006, pages 5799 - 5805

**Description**

La présente invention a pour objet une composition de bactéries ayant des propriétés d'immunomodulation, ainsi qu'un procédé pour conférer à un milieu des propriétés d'immunomodulation et également l'utilisation de cette composition. L'immunomodulation est la capacité à améliorer les fonctions immunitaires globales aussi bien dans les situations saines que pathologiques.

Parmi les bactéries, certaines ont une influence positive sur le système immunitaire du milieu intestinal, notamment les bactéries lactiques et les bifidobactéries, et sont qualifiées de bactéries ou souches « probiotiques ».

Par bactérie ou souche probiotique, on entend une souche qui ingérée vivante, exerce un effet bénéfique sur l'hôte en ayant une action sur l'équilibre de la flore intestinale et / ou sur le système immunitaire intestinal. Ces souches probiotiques ont la capacité de survivre au passage à travers la partie supérieure du tube digestif. Elles sont non pathogènes, non toxiques et exercent une action bénéfique pour la santé, d'une part via les interactions écologiques avec la flore résidente du tube digestif et d'autre part via leur capacité à influencer le système immunitaire de manière positive sur le « GALT » (tissue immunitaire associé à l'intestin). Selon la définition des probiotiques, ces bactéries lorsqu'elles sont données en nombre suffisant ont la capacité de traverser vivante tout l'intestin. Elles font alors parties de la flore résidente pendant la période d'administration. Cette colonisation (ou colonisation transitoire) permet aux bactéries probiotiques d'exercer un effet bénéfique, tel que la répression de micro-organismes potentiellement pathogènes présents dans la flore et des interactions avec le système immunitaire de l'intestin.

Les souches probiotiques les plus utilisées, notamment dans les produits laitiers, sont principalement des bactéries et des levures, des genres suivants *Lactobacillus spp., Streptococcus spp., Enterococcus spp., Bifidobacterium spp.* et *Sacharomyces spp.*. Parmi les effets probiotiques répertoriés pour ces bactéries, on peut citer par exemple l'amélioration de la tolérence au lactose, la prévention ou le traitement d'infections gastrointestinales et urogénitales, la réduction de cancers, la réduction du taux de cholestérol sanguin. Toutefois il faut mettre l'accent sur le fait que toutes les souches des genres décrits plus haut, prises individuellement, n'ont pas ces effets mais seulement certaines d'entre-elles qui doivent être soigneusement selectionnées.

Afin de répondre aux exigences des industriels il est devenu nécessaire de trouver des souches ou mélange de souches qui soient performantes et qui permettent une stimulation du système immunitaire (immunomodulation).

Aussi le problème que se propose de résoudre la présente invention est de fournir une composition de bactéries ayant des propriétés probiotiques.

La présente invention a pour but de répondre à ces exigences.

Dans ce but la présente invention propose, une composition de bactéries à propriétés d'immunomodulation qui comprend au moins une souche sélectionnée parmi le groupe constitué par *Lactobacillus plantarum* PTA-4799, *Lactobacillus salivarius* PTA-4800, *Lactobacillus paracasei* PTA-4798, *Bifidobacterium bifidum* PTA-4801, *Bifidobacterium lactis* PTA-4802 et *Bifidobacterium lactis* PTA-5658.

L'invention a également pour objet une composition alimentaire ou pharmaceutique comprenant la composition décrite ci-dessus.

L'invention décrit un procédé pour conférer à un milieu des propriétés d'immunomodulation.

L'invention a également pour autre objet l'utilisation de cette composition dans la préparation d'un support administré à l'homme ou à des animaux dans un but thérapeutique ou prophylactique au niveau du système gastro-intestinal.

La composition selon l'invention a pour avantage de proposer des vertus indiscutables et qui viennent enrichir la palette des souches disponibles.

Une telle composition est particulièrement avantageuse lorsqu'elle est administrée à l'homme ou à des animaux dans un but thérapeutique ou prophylactique au niveau du système gastra-intestinal, notamment au niveau de la réduction des réactions inflammatoires et / ou allergiques.

La présente invention a encore pour avantage de conserver toutes ses propriétés lorsqu'elle est incorporée dans un support pharmaceutiquement acceptable ou dans un produit alimentaire.

D'autres avantages et caractéristiques de la présente invention apparaîtront clairement à la lecture de la description et des exemples donnés à titre purement illustratif et non limitatif, qui vont suivre.

Tout d'abord l'invention a pour objet une composition de bactéries à propriétés d'immunomodulation qui comprend au moins une souche sélectionnée parmi le groupe constitué par *Lactobacillus plantarum* PTA-4799, *Lactobacillus salivarius* PTA-4800, *Lactobacillus paracasei* PTA-4798, *Bifidobacterium bifidum* PTA-4801, *Bifidobacterium lactis* PTA-4802 et *Bifidobacterium lactis* PTA-5658.

*Lactobacillus acidophilus* est une souche Gram-positif,

Avantageusement il s'agit d'une souche catalase négative, avec un métabolisme homofermentaire donnant lieu à la production d'acide lactique.

*Lactobacillus acidophilus* peut également produire une bactériocine, la lactacine, active contre d'autres microorganismes.

*Lactobacillus acidophilus* peut présenter une bonne résistance à la pepsine, dans des conditions de pH acide (environ au moins 73% des bactéries sont encore vivantes au bout de 40 minutes de traitement).

Plus particulièrement *Lactobacillus acidophilus* peut présenter une très bonne résistance à la pancréatine (au moins 100% des bactéries sont encore vivantes au bout de 40 minutes de traitement).

Avantageusement le *Lactobacillus acidophilus* peut présenter une bonne tolérance aux sels biliaires.

De préférence, *Lactobacillus acidophilus* peut être qualifié « d'hydrophobe » c'est à dire qui présente une forte affinité pour les solvants organiques hydrophobes polaire ou non polaire, comme par exemple le n-Décane, le chloroforme, l'hexadécane ou le xylène.

*Lactobacillus acidophilus* peut induire la production de cytokines. Cette détection de l'induction de cytokines a été réalisée grâce à un test de stimulation in vitro de cellules mononucléées isolées du sang périphérique (PBMC). Parmi les cytokines induites au cours de ce test, on peut citer l'interleukine 10 (IL10), l'interféron $\gamma$ (IFN $\gamma$) et le facteur nécrosant de tumeur $\alpha$ (TNF$\alpha$). Par contre le *Lactobacillus acidophilus* utilisé selon l'invention induit peu ou pas la sécrétion de l'interleukine 12 (IL12) en utilisant le même test.

*Lactobacillus acidophilus* peut être le *Lactobacillus acidophilus* PTA-4797. Cette souche *Lactobacillus acidophilus* a été déposée conformément au Traité de Budapest auprès de la Collection américaine des Cultures de Microorganismes où elle est enregistrée sous le numéro de dépôt PTA-4797.

La composition de bactéries probiotiques selon l'invention comprend également au moins une souche de *Lactobacillus plantarum.*

Le *Lactobacillus plantarum* utilisé selon l'invention est de préférence une souche Gram-positif. Avantageusement il s'agit d'une souche catalase négative, avec un métabolisme homofermentaire donnant lieu à la production d'acide lactique.

De préférence il s'agit d'un *Lactobacillus plantarum* résistant à la pepsine, dans des conditions de pH acide (environ au moins 95% des bactéries sont encore vivantes au bout de 40 minutes de traitement).

Plus particulièrement le *Lactobacillus plantarum* utilisé selon l'invention présente une bonne résistance à la pancréatine (environ au moins 79% des bactéries sont encore vivantes au bout de 40 minutes de traitement).

Avantageusement le *Lactobacillus plantarum* utilisé selon l'invention présente une bonne résistance aux sels biliaires.

Le *Lactobacillus plantarum* utilisé selon l'invention peut induire la production de cytokines. Cette détection de l'induction de cytokines a été réalisée grâce à un test de stimulation in vitro de cellules mononucléées isolées de sang périphériques (PBMC). Parmi les cytokines induites au cours de ce test, on peut citer l'interleukine 10 (IL10), l'interféron $\gamma$ (IFN $\gamma$) et le facteur nécrosant de tumeur $\alpha$ (TNF$\alpha$). Par contre le *Lactobacillus plantarum* utilisé selon l'invention induit peu ou pas la sécrétion de l'interleukines 12 (IL12) en utilisant le même test.

Le *Lactobacillus plantarum* utilisé selon l'invention est le *Lactobacillus plantarum* PTA-4799. Cette souche *Lactobacillus plantarum* a été déposée le conformément au Traité de Budapest auprès de la Collection américaine des Cultures de Microorganismes où elle est enregistrée sous le numéro de dépôt PTA-4799.

Une variante de la composition selon l'invention est une composition comprenant le *Lactobacillus plantarum* PTA-4799.

La composition de bactéries probiotiques selon l'invention comprend également au moins une souche de *Lactobacillus salivarius.*

Le *Lactobacillus salivarius* utilisé selon l'invention est une souche Gram-positive. Avantageusement il s'agit d'une souche catalase négative, avec un métabolisme homofermentaire donnant lieu à la production d'acide lactique.

Plus particulièrement le *Lactobacillus salivarius* utilisé selon l'invention présente une bonne résistance à la pancréatine (au moins 100% des bactéries sont toujours vivantes après 40 minutes de traitement).

Avantageusement le *Lactobacillus salivarius* utilisé selon l'invention présente une bonne tolérance aux sels biliaires.

Le *Lactobacillus salivarius* utilisé selon l'invention peut induire la production de cytokines. Cette détection de l'induction de cytokines a été réalisée grâce à un test de stimulation in vitro de cellules mononucléées isolées de sang périphérique (PBMC). Parmi les cytokines induites au cours de ce test, on peut citer l'interleukine 10 (IL10) et le facteur nécrosant de tumeur $\alpha$ (TNF$\alpha$). Par contre le *Lactobacillus salivarius* utilisé selon l'invention induit peu ou pas la sécrétion de l'interleukine 12 (IL12) et de l'interféron $\gamma$ (IFN $\gamma$) utilisant ce même test.

Le *Lactobacillus salivarius* est le *Lactobacillus salivarius* PTA-4800. Cette souche *Lactobacillus salivarius* a été déposée conformément au Traité de Budapest auprès de la Collection américaine des Cultures de Microorganismes où elle est enregistrée sous le numéro de dépôt PTA-4800.

Une variante de la composition selon l'invention est une composition comprenant le *Lactobacillus salivarius* PTA-4800.

La composition de bactéries probiotiques selon l'invention comprend également au moins une souche de *Lactobacillus paracasei.*

Le *Lactobacillus paracasei* utilisé selon l'invention est une souche Gram-positif.

Avantageusement il s'agit d'une souche catalase négative, avec un métabolisme homofermentaire donnant lieu à la production d'acide lactique.

De préférence il s'agit d'un *Lactobacillus paracasei* présentant une faible résistance à la pepsine, dans des conditions de pH acide (environ au moins 17,5% des bactéries sont encore vivantes au bout de 40 minutes de traitement).

Plus particulièrement le *Lactobacillus paracasei* utilisé selon l'invention présente une bonne résistance à la pancréatine (environ au moins 100% des bactéries sont encore vivantes au bout de 40 minutes de traitement).

Avantageusement le *Lactobacillus paracasei* utilisé selon l'invention présente une bonne résistance aux sels biliaires.

Le *Lactobacillus paracasei* utilisé selon l'invention peut induire la production de cytokines. Cette détection de l'induction de cytokines a été réalisée grâce à un test de stimulation in vitro de cellules mononucléées isolées de sang périphériques (PBMC). Parmi les cytokines induites au cours de ce test, on peut citer l'interleukine 10 (IL10), l'interféron $\gamma$ (IFN $\gamma$) et le facteur nécrosant de tumeur $\alpha$ (TNF$\alpha$). Par contre le *Lactobacillus paracasei* utilisé selon l'invention induit peu ou pas la sécrétion de l'interleukine 12 (IL12) en utilisant le même test.

Le *Lactobacillus paracasei* utilisé selon l'invention est le *Lactobacillus paracasei* PTA-4798. Cette souche *Lactobacillus paracasei* a été déposée conformément au Traité de Budapest auprès de la Collection américaine des Cultures de Microorganismes où elle est enregistrée sous le numéro de dépôt PTA-4798.

Une variante de la composition selon l'invention est une composition comprenant le *Lactobacillus paracasei* PTA-4798.

La composition de bactéries probiotiques selon l'invention comprend également au moins une souche de *Bifidobacterium bifidum.*

Le *Bifidobacterium bifidum* utilisé selon l'invention est une souche Gram-positive. Avantageusement il s'agit d'une souche catalase négative, avec un métabolisme homofermentaire donnant lieu à la production d'acide lactique et d'acide acétique.

Le *Bifidobacterium bifidum* utilisé selon l'invention peut induire la production de cytokines. Cette détection de l'induction de cytokines a été réalisée grâce à un test de stimulation in vitro de cellules mononucléées isolées de sang périphériques (PBMC). Parmi les cytokines induites au cours de ce test, on peut citer l'interleukine 10 (IL10), l'interféron $\gamma$ (IFN $\gamma$) et le facteur nécrosant de tumeur $\alpha$ (TNF$\alpha$). Par contre le *Bifidobacterium bifidum* utilisé selon l'invention induit peu ou pas la sécrétion de l'interleukine 12 (IL12) en utilisant le même test.

Le *Bifidobacterium bifidum* utilisé selon l'invention est le *Bifidobacterium bifidum* PTA-4801. Cette souche *Bifidobacterium bifidum* a été déposée conformément au Traité de Budapest auprès de la Collection américaine des Cultures de Microorganismes où elle est enregistrée sous le numéro de dépôt PTA-4801.

Une variante de la composition selon l'invention est une composition comprenant le *Bifidobacterium bifidum* PTA-4801.

La composition de bactéries probiotiques selon l'invention comprend également au moins une souche de *Bifidobacterium lactis.*

Le ou les *Bifidobacterium lactis* utilisés selon l'invention sont issus d'une souche Gram-positive. Avantageusement il s'agit d'une souche catalase négative, avec un métabolisme hétérofermentaire donnant lieu à la production d'acide lactique et d'acide acétique.

Le *Bifidobacterium lactis* utilisé selon l'invention peut induire la production de cytokines. Cette détection de l'induction de cytokines a été réalisée grâce à un test de stimulation in vitro de cellules mononucléées isolées de sang périphériques (PBMC). Parmi les cytokines induites au cours de ce test, on peut citer l'interleukine 10 (IL10), l'interféron $\gamma$ (IFN $\gamma$) et le facteur nécrosant de tumeur $\alpha$ (TNF$\alpha$). Par contre selon le *Bifidobacterium lactis* utilisé selon l'invention, celui-ci peut induire ou non la sécrétion de l'interleukine 12 (IL12) en utilisant le même test.

Le *Bifidobacterium lactis* utilisé selon l'invention est soit le *Bifidobacterium lactis* PTA-4802, soit *Bifidobacterium lactis* PTA-5658. Le *Bifidobacterium lactis* PTA-4802 a été déposé conformément au Traité de Budapest auprès de la Collection américaine des Cultures de Microorganismes où il est enregistré sous le numéro de dépôt PTA-4802.

Le *Bifidobacterium lactis* PTA-5658 a été déposé conformément au Traité de Budapest auprès de la Collection américaine des Cultures de Microorganismes où il est enregistré sous le numéro de dépôt PTA-5658.

Une variante de la composition selon l'invention est une composition comprenant le *Bifidobacterium lactis* PTA-4802.

Une autre variante de la composition selon l'invention est une composition comprenant le *Bifidobacterium lactis* PTA-5658.

Une autre variante de la composition selon l'invention est une composition de bactéries qui comprend au moins une souche sélectionnée parmi le groupe constitué par *Lactobacillus plantarum* PTA-4799, et *Bifidobacterium lactis* PTA-4802.

Plus particulièrement, la composition selon l'invention peut comprendre au moins 2 souches sélectionnées parmi le groupe constitué par *Lactobacillus acidophilus* PTA-4797, *Lactobacillus plantarum* PTA-4799, *Lactobacillus*

*salivarius* PTA-4800, *Lactobacillus paracasei* PTA-4798, *Bifidobacterium bifidum* PTA-4801, *Bifidobacterium lacis* PTA-4802 et *Bifidobacterium lactis* PTA-5658.

Plus particulièrement, la composition selon l'invention peut comprendre au moins 3 souches sélectionnées parmi le groupe constitué par *Lactobacillus acidophilus* PTA-4797, *Lactobacillus plantarum* PTA-4799, *Lactobacillus salivarius* PTA-4800, Lactobacillus *paracasei* PTA-4798, *Bifidobacterium bifidum* PTA-4801, *Bifidobacterium lactis* PTA-4802 et *Bifidobacterium lactis* PTA-5658.

Plus particulièrement, la composition selon l'invention peut comprendre de préférence un mélange de *Lactobacillus salivarius* PTA-4800 avec une souche du genre *Bifidobacterium, choisi parmi Bifidobacterium bifidum* PTA-4801, *Bifidobacterium lactis* PTA-4802 ou *Bifidobacterium lactis* PTA-5658.

Plus particulièrement, la composition selon l'invention peut comprendre de préférence un mélange de *Lactobacillus acidophilus* PTA-4797 avec une souche du genre *Bifidobacterium, choisi parmi Bifidobacterium bifidum* PTA-4801, *Bifidobacterium lactis* PTA-4802 ou *Bifidobacterium lactis* PTA-5658.

Plus particulièrement, la composition selon l'invention peut comprendre de préférence un mélange de *Lactobacillus plantarum* PTA-4799 avec une souche du genre *Bifidobacterium, choisi parmi Bifidobacterium bifidum* PTA-4801, *Bifidobacterium lactis* PTA-4802 ou *Bifidobacterium lactis* PTA-5658.

De préférence la composition selon l'invention peut comprendre un mélange de *Lactobacillus acidophilus* PTA-4797, *Lactobacillus plantarum* PTA-4799, *Lactobacillus salivarius* PTA-4800, *Lactobacillus paracasei* PTA-4798, *Bifidobacterium bifidum* PTA-4801, *Bifidobacterium lactis* PTA-4802 et *Bifidobacterium lactis* PTA-5658.

La composition selon l'invention peut être administrée sous forme d'une suspension bactérienne, avant ou après congélation, ou d'une poudre lyophilisée. En effet quelle que soit la forme utilisée, la composition peut être congelée.

La proportion relative de chaque bactéries dans la composition peut varier dans de large limite par exemple de 99 / 1 à 1 / 99 dans le cas où il y a au moins deux souches.

La composition selon l'invention peut comprendre de $10^6$ à $10^{11}$ CFU de bactéries / g de composition, et plus particulièrement de $10^8$ à $10^{11}$ CFU de bactéries / g de composition. Le terme CFU signifie « colony forming units » selon l'expression technique anglaise. Par gramme de composition, on entend de préférence le produit alimentaire ou la préparation pharmaceutique, et de préférence $10^9$ à $10^{11}$ CFU / g pour la forme lyophilisée.

La composition selon l'invention est utile notamment pour le traitement, la prévention primaire ou de récidive, ainsi que pour la prévention et / ou la réduction de maladie de l'inflammation de l'intestin.

La composition selon l'invention est utile notamment pour le maintien de l'homéostasie du systeme immunitaire.

La composition selon l'invention est également utile pour la prévention et / ou la réduction de l'allergénicité.

On peut aussi noter que la composition selon l'invention est utile comme immunoadjuvant.

L'invention a aussi pour objet une souche sélectionnée parmi le groupe constitué par *Lactobacillus plantarum* PTA-4799, *Lactobacillus salivarius* PTA-4800, *Lactobacillus paracasei* PTA-4798, *Bifidobacterium bifidum* PTA-4801, *Bifidobacterium lactis* PTA-4802 et *Bifidobacterium* lactis PTA-5658 pour conférer à un milieu des propriétés d'immunomodulation.

La composition selon l'invention peut être utilisée sous le forme d'un produit alimentaire ou d'une préparation pharmaceutique.

L'invention a également pour objet une composition alimentaire ou pharmaceutique comprenant la composition décrite ci-dessus.

Par composition pharmaceutique, on entend entre autre une composition sous forme de comprimés ou de tablettes.

De préférence la composition alimentaire comprenant la composition selon l'invention est un complément alimentaire, une boisson ou une poudre à base de lait.

Plus préférentiellement, la composition alimentaire comprenant la composition selon l'invention est un produit laitier.

Encore plus préférentiellement la composition alimentaire comprenant la composition selon l'invention est un lait maternisé.

La composition alimentaire ou pharmaceutique selon l'invention peut avoir des propriétés d'immunomodulation.

L'invention a également pour autre objet l'utilisation de cette composition dans la préparation d'un support administré à l'homme ou à des animaux dans un but thérapeutique ou prophylactique au niveau du système gastro-intestinal.

L'invention a également pour objet une composition pharmaceutique comprenant la composition de bactéries décrite ci-dessus utile pour la prévention de la maladie de l'inflammation de l'intestin.

L'invention a également pour objet une composition pharmaceutique comprenant la composition de bactéries décrite ci-dessus utile pour l'immunomodulation.

Les figures 1, 3, et 5 représentent des protocoles d'injection,

Les figures 2, 4, 6, 7 et 8 représentent les scores de Wallace et d'Ameho, ainsi que la perte de poids.

Des exemples concrets mais non limitatifs de l'invention vont maintenant être décrits.

**EXEMPLES**

**1/ Test PBMC :**

Les cellules mononucléées isolées de sang périphérique (PBMC) sont préparées par centrifugation de sang humain, dérivé de donneurs connus et sont ensuite purifiées sur un gradient de Ficoll. Les cellules sont récoltées, lavées, les globules rouges sont enlevés, et les cellules sont comptées.

Les bactéries sont préparées selon des conditions standard et comptées en étalant sur un milieu agarose différentes dilutions ($10^{-7}$ ; $10^{-8}$ ; $10^{-9}$ dans une solution de Ringer).

Les cellules sont lavées 3 fois et remise en suspension dans un tampon de sels de phosphate.

Les cellules PBMC sont stimulées pendant 48 heures avec les bactéries (le contrôle négatif (témoin) est une stimulation avec un tampon de sels de phosphate) sous des conditions appropriées de $CO_2$. Puis le surnageant contenant les cytokines est congelé à moins 20°C.

Les taux d'expression des cytokines sont déterminés par des tests ELISA (« Enzyme linked immuno sorbent assay »). Les plaques ELISA sont recouvertes d'un anticorps anti-cytokine (pendant une nuit) et l'anticorps est fixé avec un détergent, tel que le tween 80.

Une gamme standart est préparée pour des concentrations en cytokines connues qui couvre une zone de détection de 15,62 à 2000 pg/ml (incubation toute la nuit).

La détection des cytokines et leur quantification est réalisée avec une réaction à la streptavidine sur le substrat (10 mg d'ABTS/10ml d'acide citrique dans un tampon 0,1 M, pH4,35/20 $\mu$l $H_2O_2$).

Les cytokines sont soit pro-inflamatoires / Th1 (TNF$\alpha$, IFN$\gamma$, IL12) soit anti-inflammatoires (IL10).

| Souches de bactéries | IL-10 | IL-12 | IFN-$\gamma$ | TNF-$\alpha$ |
|---|---|---|---|---|
| **L. acidophilus PTA-4797** | 309 | | 1970 | 27591 |
| **L. salivarius PTA-4800** | 6881 | 31 | 1148 | 23509 |
| **L. paracasei PTA-4798** | 300 | 31 | 921 | 14046 |
| **B. bifidum PTA-4801** | 576 | 88 | 10490 | 100700 |
| **B. lactis PTA-4802** | 506 | 676 | 11925 | 263200 |
| **B. lactis PTA-5658** | 1081 | 65 | 10257 | 159700 |
| **L. plantarum PTA-4799** | 318 | | 21877 | 21877 |
| **témoin** | 31 | 31 | 31 | 31 |
| Les valeurs du tableau sont en picogrammes / ml | | | | |

**2/ Préparation de divers compositions à partir de *Lactobacillus acidophilus* PTA-4797, *Lactobacillus plantarum* PTA-4799, *Lactobacillus salivarius PTA-4800,* de *Bifidobacterium bifidum* PTA-4801, de *Bifidobacterium lactis* PTA-5658 et de *Bifidobacterium lactis* PTA-4802 :**

2-1/ Un mélange de 3 souches est préparé contenant les souches *Lactobacillus acidophilus* PTA-4797, *Lactobâcillus plantarum* PTA-4799, et *Bifidobacterium lactis* PTA-4802. Ce mélange est appelé LAB.

Pour montrer in vivo, la capacité d'immunomodulation de ce mélange, des tests animaux sont réalisés dans le but de diminuer l'inflammation intestinale induite chimiquement chez des souris. Plusieurs modèles permettent de mimer la maladie inflammatoire humaine de l'intestin.

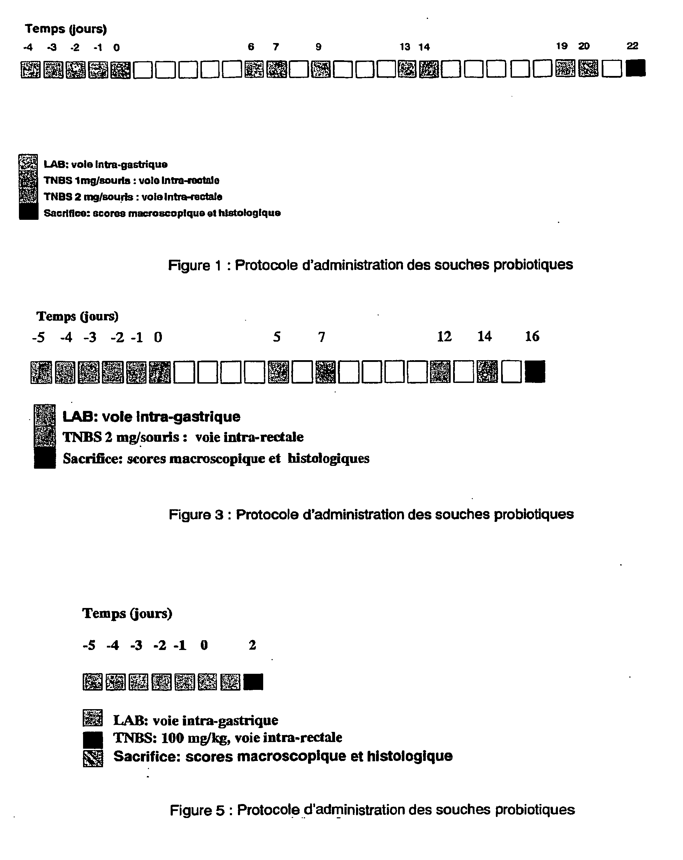

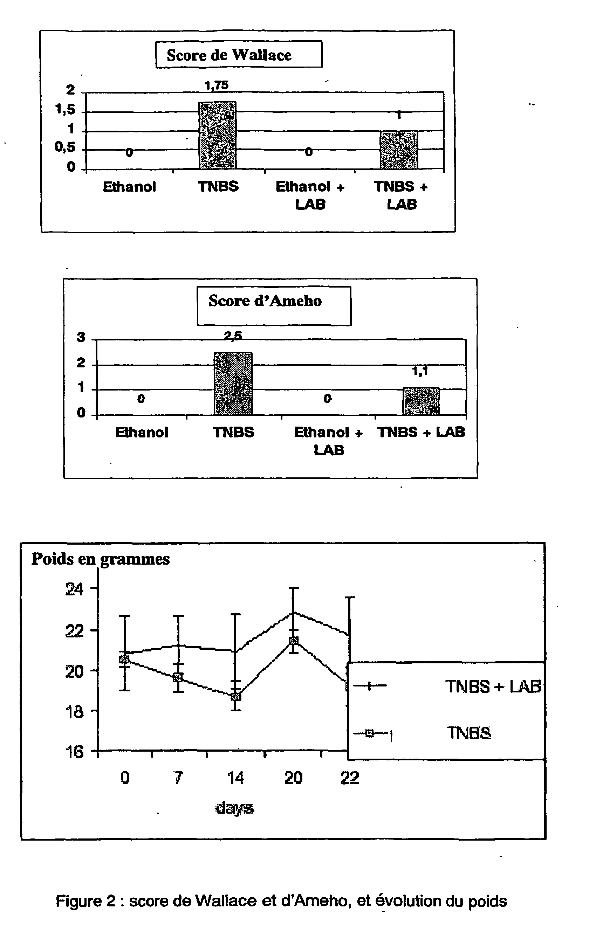

Dans un premier modèle, des injections de 1 mg/souris de TNBS (l'acide trinitrobenzène sulfonique qui est un agent induisant la colite) aux jours 0, 7 et 14, et 2 mg/souris de TNBS au jour 20 (double dose) provoquent une colite chronique. Le protocole d'injection est shématisé à la figure 1. La prise de bactéries (LAB) aux jours - 4 à-1, 6, 9, 13 et 19 (figure 1) montre des effets macroscopiques et histologiques, qui sont évalués au jour 22 selon les méthodes standard (Wallace et Ameho respectivement) (figure 2).

Les résultats obtenus (voir figure 2) montrent clairement l'amélioration des symptômes de la colite par:

- une diminution de l'augmentation de la muqueuse
- une diminution de l'inflammation
- perte de poids

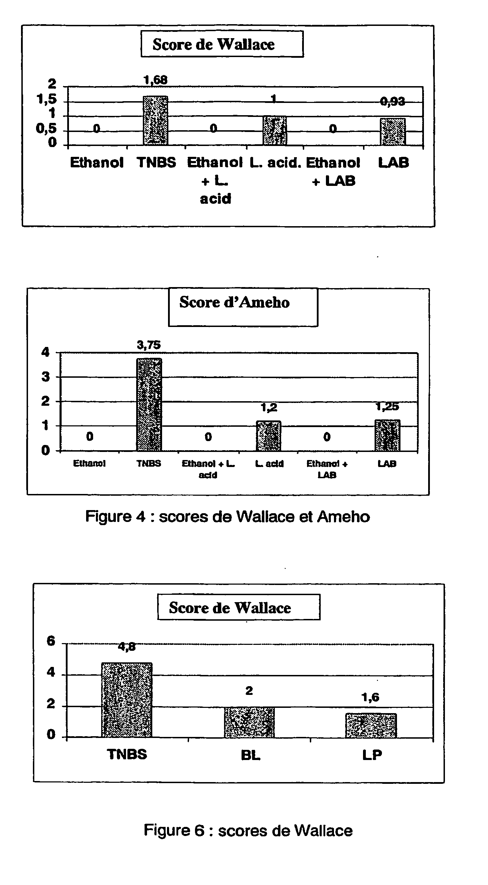

2-2/ Un second modèle de colite chronique a été aussi utilisé (basé sur Camoglio et al., Eur J Immunol 2000), 2 mg/souris de TNBS injecté intra-rectalement aux jours 0, 7 et 14 déclenchent une colite chronique grave. Le protocole d'injection est shématisé à la figure 3. La prise de bactéries, décrites au point 2-1 (LAB), aux jours -5 à -1, aux jours 5 et 12 (figure 3) montre des effets macroscopiques et histologiques, qui sont évalués au jour 16 selon les méthodes standarts (Wallace et Ameho respectivement) (figure 4).

Les résultats obtenus (voir figure 4) montrent clairement l'amélioration des symptômes de la colite par :

- l'absence de lésions nécrotiques
- une diminution de l'augmentation de la muqueuse
- une diminution de l'inflammation

2-3/ Un troisième modèle aigu de colite chez la souris a été aussi réalisé. 100 mg de TNBS/kg de souris est injecté au jour 0. Cela provoque une colite aigue. Le protocole d'injection est shématisé à la figure 5. La prise d'une souche individuelle de bactéries ou de mélanges de bactéries ($10^8$ bactéries / souris) au jour -5 à 0 montre des effets macroscopiques et histologiques, qui sont évalués au jour 2 (figure 6, 7 et 8).

La figure 6 montre les scores de Wallace obtenus lorsqu'une lorsqu'une souche individuelle de *Bifidobacterium lactis* PTA-4802 ou de *Lactobacillus plantarum* PTA-4799 est injectée aux souris.

La figure 7 montre les scores de Wallace obtenus lorsqu'une souche individuelle de *Lactobacillus salivarius PTA-4800 (A), ou* de *Bifidobacterium bifidum* PTA-4801 (B), ou de *Bifidobacterium lactis* PTA-4802 (C), ou de *Bifidobacterium lactis* PTA-5658 (D) est injectée aux souris. Le témoin (T) correspond à l'injection de TNBS en absence de bactéries.

La figure 8 montre les scores de Wallace obtenus lorsqu'un mélange de bactéries est injecté aux souris. Le mélange comprend différentes souches de bactéries suivantes :

Mélange 1 : *Lactobacillus acidophilus* PTA-4797 et *Bifidobacterium lactis* PTA-4802 à parts égales ;
Mélange 2 : *Bifidobacterium lactis* PTA-4802 et *Bifidobacterium bifidum* PTA-4801 à parts égales ;
Mélange 3 : 40% *Bifidobacterium lactis* PTA-4802 et 40% *Bifidobacterium bifidum* PTA-4801 et 20% *Lactobacillus salivarius* PTA-4800 ;
Mélange 4 : 25% *Bifidobacterium lactis* PTA-4802 et 25% *Bifidobacterium bifidum* PTA-4801 et 16,66% *Lactobacillus salivarius* PTA-4800 et 16,66% *Lactobacillus acidophilus* PTA-4797 et 16,66% *Lactobacillus plantarum* PTA-4799 ;
Mélange 5 : 40% *Bifidobacterium lactis* PTA-4802 et 40% *Bifidobacterium bifidum* PTA-4801 et 6,66% *Lactobacillus salivarius* PTA-4800 et 6,66% *Lactobacillus acidophilus* PTA-4797 et 6,66% *Lactobacillus plantarum* PTA-4799.

Ces pourcentages sont exprimés en poids.
Les résultats obtenus dans les mélanges 2, 3 et 5 sont significativement différents des résultats du témoin.

Une amélioration macroscopique claire des symptômes de la colite aigüe sont mis en évidence pour les souches individuelle de bactéries ou de mélanges de bactéries (figure 6, 7 et 8).

2-4/ En général tous les modèles de colites induites par le TNBS chez la souris montrent que l'apport de bactéries selon l'invention à ces souris reduit significativement le niveau de translocation intestinale des bactéries indigènes de la flore intestinale vers les ganglions lymphatiques mésentériques et la rate après l'induction de la colite. De plus, aucune translocation des souches de bactéries selon l'invention n'est observée.

## 3/ Résistance à la pepsine

Le but de ce test est d'évaluer la résistance des bactéries au passage de la barrière gastrique en déterminant le nombre de bactéries qui survivent après mise en culture en présence d'une solution de pepsine dans un environnement acide. 100 $\mu$l d'une culture stock congelée à -80°C est inoculée dans 10 ml de milieu de culture MRS (Man Rogosa Sharp) et est incubée toute la nuit à 30°C ou 37°C. Puis les cellules sont lavées 3 fois dans un tampon de sels de phosphate à pH7 et le culot est resuspendu dans 1 ml de tampon de sels de phosphate et inoculé en aliquot de 200 $\mu$l dans 4 tubes avec 1 ml d'une solution à pH 2 de pepsine filtrée supplémentée avec 300 $\mu$l de NaCl (0,5%). Le comptage des cellules est réalisé sur des aliquots de 100 $\mu$l prélevés des tubes incubés à t=0 (temps d'inoculation), t=0 + 5 minutes, t=0 + 20 minutes, t=0 + 40 minutes, t=0 + 60 minutes et 100 $\mu$l d'une dilution à $10^{-5}$, $10^{-6}$ et $10^{-7}$ sont étalés sur gélose (dilutions faites 10 fois consécutivement dans 1 ml de solution de Ringer). Le pourcentage de bactéries survivantes est calculé pour chaque temps d'incubation par lecture des colonies issues de ces dilutions sucessives.

Tous les résultats montrés dans les tableaux sont le résultat d'expériences faites en triple.

| Souches | 5 min | 20 min | 40 min | 60 min |
|---|---|---|---|---|
| L. acidophilus PTA-4797 | 91 | 74 | 73 | 55 |
| L.plantarum PTA-4799 | 100 | 100 | 95 | 42 |
| L. salivarius PTA-4800 | 100 | 66 | 23 | 7 |
| L. paracasei PTA-4798 | 100 | 17,5 | 17,5 | 1,5 |
| Les résultats sont le pourcentage de survie comparé à T=0. | | | | |

**4/ Résistance à la pancréatine**

Le but de ce test est d'évaluer la résistance des bactéries au passage de la barrière gastrique en déterminant le nombre de bactéries qui survivent après mise en culture en présence d'une solution de pancréatine dans un environnement acide. 100 $\mu$l d'une culture stock congelée à -80°C est inoculée dans 10 ml de milieu de culture MRS (Man Rogosa Sharp) et est incubée toute la nuit à 30°C ou 37°C. Puis les cellules sont lavées 3 fois dans un tampon de sels de phosphate à pH7 et le culot est resuspendu dans 1 ml de tampon de sels de phosphate et inoculé en aliquot de 200 $\mu$l dans 4 tubes avec 1 ml d'une solution à pH 8 de pancréatine filtrée supplémentée avec 300 $\mu$l de NaCl (0,5%). Le comptage des cellules est réalisé sur des aliquots de 100 $\mu$l prélevés des tubes incubés à t=0 (temps d'inoculation), t=0 + 5 minutes, t=0 + 20 minutes, t=0 + 40 minutes, t=0 + 60 minutes, t=0 + 120 minutes et 100 $\mu$l d'une dilution à $10^{-5}$, $10^{-6}$ et $10^{-7}$ sont étalées sur gélose (dilutions faites 10 fois consécutivement dans 1 ml de solution de Ringer). Le pourcentage de bactéries survivantes est calculé pour chaque temps d'incubation par lecture des colonies issues de ces dilutions successives.

Tous les résultats montrés dans les tableaux sont le résultat d'expériences faites en triple.

| Souches | 5 min | 20 min | 40 min | 60 min | 120 min |
|---|---|---|---|---|---|
| L. acidophilus PTA-4797 | 83 | 100 | 100 | 170 | 170 |
| L. salivarius PTA-4800 | 100 | 115 | 152 | 158 | 155 |
| L. plantarum PTA-4799 | 86 | 72 | 79 | 84 | 87 |
| L. paracasei PTA-4798 | 65 | 100 | 100 | 100 | 100 |
| Les résultats sont le pourcentage de survie comparé à T=0. | | | | | |

**5/ Résistance aux sels biliaires**

La procédure expérimentale est la suivante. 100 $\mu$l d'une culture stock congelée à -80°C est inoculée dans 10 ml de milieu de culture MRS (Man Rogosa Sharp) et est incubée toute la nuit à 30°C ou 37°C. La densité optique (DO) est mesurée et une dilution est préparée (DO=0,05 à 0,1) dans 2 flacons. L'un des flacons de culture est suplémenté avec 0,3% de solution de sels biliaires (750 $\mu$l d'une solution à 12 % filtrée) et est incubé à la bonne température. La DO est régulièrement mesurée jusqu'à ce qu'une DO de 0,3 soit atteinte dans le flacon contrôle sans sels biliaires. A ce moment, la DO du flacon supplémenté est mesurée et commence une mesure du temps nécessaire pour obtenir une DO de 0,3. La résistance de la souche est ainsi évaluée comme étant le temps de croissance. Les resultats sont exprimés par un code : sensible (0 ; plus de 60 minutes), tolérante (1 ; entre 15 et 60 minutes) ou résistante (2 ; moins de 15 minutes).

| Souches | Resistance |
|---|---|
| L. acidophilus PTA-4797 | 1 |
| L.plantarum PTA-4799 | 2 |
| L. paracasei PTA-4798 | 2 |
| L. salivarius PTA-4800 | 1 |

**6/ Hydrophobicité et affinité pour les solvants organiques**

Le test MATS décrit par Rosdenberg et al., 1980 (FEMS Microbiol. Letters 9 ; 29-33) a été utilisé. En fait, le test mesure (croissance toute la nuit sous des conditions normales in vitro et double lavage avec un tampon de sels de phosphate) l'hydrophobicité et la polarité de la paroi des cellules des bactéries, basée sur la répartition (mesurée par densité optique) des bactéries en 2 phases liquides aqueuses non mélangeable et 5 solvants respectifs. Le milieu de croissance est le MRS (Man Rogosa Sharp) et la phase aqueuse est un tampon du type tampon de sels de phosphate. Les solvants sont le décane, l'hexadécane, acétate d'éthyl, le chloroforme et le xylène.

L'affinité pour le chloroforme, solvant acide, reflète la nature de donneur d'électrons de la bactérie (réaction de base).

L'affinité pour l'acétate d'éthyl, solvant basique, reflète la nature d'accepteur d'électrons de la bactérie (réaction acide).

L'affinité pour les solvants apolaires (décane, hexadécane, xylène) reflète le caractère hydrophobe de la bactérie.

La possibilité d'une haute hydrophobicité est corrélée à la présence de glycoprotéines sur la surface des parois des cellules, pendant qu'une faible hydrophobicité peut être liée à la présence de polysaccharides à la surface de la paroi des cellules.

$$\% \text{ D'HYDROPHOBICITE} = (1 - A/A_0) \times 100 \ ;$$

avec
- $A_0$: DO d'origne à 540 nm (soit plus ou moins à DO = 0,6 dans un tampon de sels de phosphate)
- A: DO de la phase aqueuse (pour 1 ml) après mélange au vortex et une période de stabilisation

| Souches | décane | hexadecane | acétate d'éthyl | chloroforme | xylene |
|---|---|---|---|---|---|
| L. acidophilus PTA-4797 | 81.9 | 60.4 | 44.2 | 90.3 | 90.9 |
| L. salivarius PTA-4800 | 23.02 | 38.17 | 2.25 | 79.28 | 51.18 |
| L. paracasei PTA-4798 | 36.5 | 37.4 | 28.8 | 45.2 | 30.6 |
| L. plantarum PTA-4799 | 25.3 | 25.6 | 28.7 | 28.8 | 21.9 |

**Revendications**

1. Composition de bactéries à propriétés d'immunomodulation qui comprend au moins une souche sélectionnée parmi le groupe constitué par *Lactobacillus plantarum* PTA-4799, *Lactobacillus salivarius* PTA-4800, *Lactobacillus paracasei* PTA-4798, *Bifidobacterium bifidum* PTA-4801, *Bifidobacterium lactis* PTA-4802 et *Bifidobacterium lactis* PTA-5658.

2. Composition de bactéries selon la revendication 1 **caractérisée en ce que** la souche est sélectionnée parmi le groupe constitué par *Lactobacillus plantarum* PTA-4799 et *Bifidobacterium lactis* PTA-4802.

3. Composition de bactéries selon la revendication 1 **caractérisée en ce que** la souche est *Lactobacillus plantarum* PTA-4799.

4. Composition selon la revendication 1 **caractérisée en ce que** la souche est *Bifidobacterium lactis* PTA-4802 et *Bifidobacterium lactis* PTA-5658.

5. Composition de bactéries selon la revendication 1 qui comprend au moins deux souches sélectionnées parmi le groupe constitué par *Lactobacillus acidophilus* PTA-4797, *Lactobacillus plantarum* PTA-4799, *Lactobacillus salivarius* PTA-4800, *Lactobacillus paracasei* PTA-4798, *Bifidobacterium bifidum* PTA-4801, *Bifidobacterium lactis* PTA-4802 et *Bifidobacterium lactis* PTA-5658.

6. Composition de bactéries selon la revendication 1 qui comprend au moins trois souches sélectionnées parmi le groupe constitué par *Lactobacillus acidophilus* PTA-4797, *Lactobacillus plantarum* PTA-4799, *Lactobacillus salivarius* PTA-4800, *Lactobacillus paracasei* PTA-4798, *Bifidobacterium bifidum* PTA-4801, *Bifidobacterium lactis* PTA-4802 et *Bifidobacterium lactis* PTA-5658.

7. Composition de bactéries selon la revendication 1 qui comprend un mélange de *Lactobacillus acidophilus* PTA-4797, *Lactobacillus plantarum* PTA-4799, *Lactobacillus salivarius* PTA-4800, *Lactobacillus paracasei* PTA-4798, *Bifidobacterium bifidum* PTA-4801, *Bifidobacterium lactis* PTA-4802 et *Bifidobacterium lactis* PTA-5658.

8. Composition selon l'une quelconque des revendications précédentes, **caractérisée en ce qu'**elle comprend de $10^6$ à $10^{11}$ CFU de bactéries/ g de composition.

9. Composition selon l'une quelconque des revendications précédentes, **caractérisée en ce qu'**elle comprend de $10^8$ à $10^{11}$ CFU de bactéries / g de composition.

10. Composition selon l'une quelconque des revendications précédentes, **caractérisée en ce qu'**elle est utile pour la prévention et / ou la réduction de la maladie de l'inflammation de l'intestin.

11. Composition selon l'une quelconque des revendications précédentes, **caractérisée en ce qu'**elle est utile pour le maintien de l'homéostasie du système immunitaire.

12. Composition selon l'une quelconque des revendications précédentes, **caractérisée en ce qu'**elle est utile pour la prévention et / ou la réduction de l'allergénicité.

13. Composition selon l'une quelconque des revendications précédentes, **caractérisée en ce qu'**elle est utile comme immunoadjuvent.

14. Souche sélectionnée parmi le groupe constitué par *Lactobacillus plantarum* PTA-4799, *Lactobacillus salivarius* PTA-4800, *Lactobacillus paracasei* PTA-4798, *Bifidobacterium bifidum* PTA-4801, *Bifidobacterium lactis* PTA-4802 et *Bifidobacterium lactis* PTA-5658, pour son utilisation dans l'immunomodulation.

15. Composition alimentaire ou pharmaceutique comprenant la composition selon l'une des revendications 1 à 13.

16. Composition selon la revendication 15 **caractérisée en ce qu'**il s'agit d'un produit laitier.

17. Composition selon la revendication 15 ou 16 **caractérisée en ce qu'**il s'agit d'un lait maternisé.

18. Composition selon l'une quelconque des revendications 15 à 17, **caractérisée en ce que** la composition a des propriétés d'immunomodulation.

19. Composition pharmaceutique comprenant la composition selon les revendications 1 à 13 utile pour la prévention de la maladie de l'inflammation de l'intestin.

20. Composition pharmaceutique comprenant la composition de bactéries selon les revendications 1 à 13 utile pour l'immunomodulation.

21. Utilisation de la composition de la revendication 1 dans la préparation d'un support administrée à l'homme ou à des animaux dans un but thérapeutique ou prophylactique au niveau du système gastro-intestinal.

**Patentansprüche**

1. Bakterielle Zusammensetzung mit Immunmodulationseigenschaften, die wenigstens einen Stamm umfasst, der aus der Gruppe bestehend aus *Lactobacillus plantarum* PTA-4799, *Lactobacillus galivarius* PTA-4800, *Lactobacillus paracasei* PTA-4798, *Bifidobacterium bifidum* PTA-4801, *Bifidobacterium lactis* PTA-4802 und *Bifidobacterium lactis* PTA-5658 ausgewählt ist.

2. Bakterielle Zusammensetzung nach Anspruch 1, wobei der Stamm aus der Gruppe bestehend aus *Lactobacillus plantarum* PTA-4799 und *Bifidobacterium lactis* PTA-4802 ausgewählt ist.

**3.** Bakterielle Zusammensetzung nach Anspruch 1, wobei der Stamm *Lactobacillus plantarum* PTA-4799 ist.

**4.** Bakterielle Zusammensetzung nach Anspruch 1, wobei der Stamm *Bifidobacterium lactis* PTA-4802 oder *Bifidobacterium lactis* PTA-5658 ist.

**5.** Bakterielle Zusammensetzung nach Anspruch 1, die wenigstens zwei Stämme umfasst, die aus der Gruppe bestehend aus *Lactobacillus acidophilus* PTA-4797, *Lactobacillus plantarum* PTA-4799, *Lactobacillus salivarius* PTA-4800, *Lactobacillus paracasei* PTA-4798, *Bifidobacterium bifidum PTA-4801, Bifidobacterium lactis* PTA-4802 und *Bifidobacterium lactis* PTA-5658 ausgewählt sind.

**6.** Bakterielle Zusammensetzung nach Anspruch 1, die wenigstens drei Stämme umfasst, die aus der Gruppe bestehend aus *Lactobacillus acidophilus* PTA-4797, *Lactobacillus plantarum* PTA-4799, *Lactobacillus salivarius* PTA-4800, *Lactobacillus paracasei* PTA-4798, *Bifidobacterium bifidum* PTA-4801, *Bifidobacterium lactis* PTA-4802 und *Bifidobacterium lactid* PTA-5658 ausgewählt sind.

**7.** Bakterielle Zusammensetzung nach Anspruch 1, die eine Mischung aus *Lactobacillus acidophilus* PTA-4797, *Lactobacillus plantarum* PTA-4799, *Lactobacillus salivarius* PTA-4800, *Lactobacillus paracasei* PTA-4798, *Bifidobacterium bifidum* PTA-4801, *Bifidobacterium lactis* PTA-4802 und *Bifidobacterium lactis* PTA-5658 umfasst.

**8.** Bakterielle Zusammensetzung nach einem der vorherigen Ansprüche, **dadurch gekennzeichnet, dass** sie $10^6$ bis $10^{11}$ CFU Bakterien pro Gramm der Zusammensetzung umfasst.

**9.** Bakterielle Zusammensetzung nach einem der vorherigen Ansprüche, **dadurch gekennzeichnet, dass** sie $10^8$ bis $10^{11}$ CFU Bakterien pro Gramm der Zusammensetzung umfasst.

**10.** Bakterielle Zusammensetzung, nach einem der vorherigen Ansprüche, **dadurch gekennzeichnet, dass** die Zusammensetzung zur Verhütung und/oder Reduzierung von entzündlicher Darmerkrankung nützlich ist.

**11.** Bakterielle Zusammensetzung nach einem der vorherigen Ansprüche, **dadurch gekennzeichnet, dass** die Zusammensetzung zur Aufrechterhaltung der Homöostase des Immunsystems nützlich ist.

**12.** Bakterielle Zusammensetzung nach einem der vorherigen Ansprüche, **dadurch gekennzeichnet, dass** die Zusammensetzung zur Verhütung und/oder Reduzierung von Allergenität nützlich ist.

**13.** Bakterielle Zusammensetzung nach einem der vorherigen Ansprüche, **dadurch gekennzeichnet, dass** die Zusammensetzung als Immunadjuvans nützlich ist.

**14.** Stamm, der aus der Gruppe bestehend aus *Lactobacillus plantarum* PTA-4799, *Lactobacillus salivarius* PTA-4800, *Lactobacillus paracasei* PTA-4798, *Bifidobacterium bifidum* PTA-4801, *Bifidobacterium lactis* PTA-4802 und *Bifidobacterium lactis* PTA-5658 zur Verwendung bei Immunmodulation ausgewählt ist.

**15.** Lebensmittel oder pharmazeutische Zusammensetzung, das/die eine Zusammensetzung nach einem der Ansprüche 1 bis 13 umfasst.

**16.** Zusammensetzung nach Anspruch 15, **dadurch gekennzeichnet, dass** sie ein Milchprodukt ist.

**17.** Zusammensetzung nach Anspruch 15 oder 16, **dadurch gekennzeichnet, dass** sie eine Säuglingsnahrung ist.

**18.** Zusammensetzung nach einem der Ansprüche 15 bis 17, wobei die Zusammensetzung Immunmodulationseigenschaften hat.

**19.** Pharmazeutische Zusammensetzung, die die Zusammensetzung nach einem der Ansprüche 1 bis 13 umfasst, die für die Verhütung von entzündlicher Darmerkrankung nützlich ist.

**20.** Pharmazeutische Zusammensetzung, die die Zusammensetzung nach einem der Ansprüche 1 bis 13 umfasst, die für Immunmodulation nützlich ist.

**21.** Verwendung der Zusammensetzung nach Anspruch 1 bei der Herstellung eines Trägers, der einem Menschen oder Tieren für einen therapeutischen oder prophylaktischen Zweck im Magen-Darm-System verabreicht wird.

**Claims**

1. A bacterial composition having immunomodulation properties comprising at least one strain selected from the group consisting of *Lactobacillus plantarum* PTA-4799, *Lactobacillus salivarius* PTA-4800, *Lactobacillus paracasei* PTA-4798, *Bifidobaclerium bifidum* PTA-4801, *Bifidobucterium lactis* PTA-4802 and *Bifidobacterium lactis* PTA-5658.

2. The bacterial composition according to claim 1, wherein the strain is selected from the group consisting of *Lactobacillus plantarum* PTA-4799, and *Bifidobacterium lactis* PTA-4802.

3. The bacterial composition according to claim 1 wherein the strain is *Lactobacillus plantarum* PTA-4799.

4. The bacterial composition according to claim 1 wherein the strain is *Bifidobacterium lactis* PTA-4802 or *Bificdobacterium lactis* PTA-5658.

5. The bacterial composition according to claim 1 comprising at least two strains selected from the group consisting of *Lactobacillus acidophilus* PTA-4797, *Luctobacillus plantarum* PTA-4799, *Lactobacillus salivarius* PTA-4800, *Lactobacillus paracasei* PTA-4798, *Bifidobacterium bifidum* PTA-4801, *Bifidobacterium lactis* PTA-4802 and *Bifidobacterium lactis* PTA-5658.

6. The bacterial composition according to claim 1 comprising at least three strains selected from the group consisting of *Lactobacillus acidophilus* PTA-4797, *Lactobacillus plantarum* PTA-4799, *Lactobacillus salivarius* PTA-4800, *Lactobacillus paracasei* PTA-4798, *Bifidobacterium bifidum* PTA-4801, *Bifidobacterium lactis* PTA-4802 and *Bifidobacterium lactis* PTA-5658.

7. The bacterial composition according to claim 1 comprising a blend of *Lactobacillus acidophilus* PTA-4797, *Lactobacillus plantarum* PTA-4799, *Lactobacillus salivarius* PTA-4800, *Lactobacillus paracasei* PTA-4798, *Bifidobacterium bifidum* PTA-4801, *Bifidobacterium lactis* PTA-4802 *and Bifidobacterium lactis* PTA-5658.

8. The bacterial composition according to any one of the preceding claims, **characterized in that** it comprises from $10^6$ to $10^{11}$ CFU of bacteria/g of composition.

9. The bacterial composition according to any one of the preceding claims, **characterized in that** it comprises from $10^8$ to $10^{11}$ CFU of bacteria/g of composition.

10. The bacterial composition according to any one of the preceding claims, **characterized in that** the composition is useful for the prevention and/or the reduction of inflammatory bowel disease.

11. The bacterial composition according to any one of the preceding claims, **characterized in that** the composition is useful for maintaining the homeostasis of the immune system.

12. The bacterial composition according to any one of the preceding claims, **characterized in that** the composition is useful for the prevention and/or the reduction of allergenicity.

13. The bacterial composition according to any one of the preceding claims, **characterized in that** the composition is useful as immunoadjuvent.

14. A strain selected from the group consisting of *Lactobacillus plantarum* PTA-4799, *Lactobacillus salivarius* PTA-4800, *Lactobacillus paracasei* PTA-4798, *Bifidobacterium bifidum* PTA-4801, *Bifidobacterium lactis* PTA-4802 and *Bifidobacterium lactis* PTA-5658 for use in immunomodulation.

15. Food or Pharmaceutical composition comprising the composition according to anyone of claims 1 to 13.

16. Composition according to claim 15, **characterized in that** it is a dairy product.

17. Composition according to claim 15 or 16, **characterized in that** it is an infant formula.

**18.** Composition according to anyone of claims 15 to 17, wherein the composition has immunomodulation properties.

**19.** Pharmaceutical composition comprising the composition according to any one of claims 1 to 13 useful for the prevention of inflammatory bowel disease.

**20.** Pharmaceutical composition comprising the composition according to any one of claims 1 to 13 useful for immunomodulation.

**21.** Use of the composition according to claim 1 in the preparation of a carrier administered to human or to animals for a therapeutic or prophylactic purpose in the gastrointestinal system.

**Temps (jours)**

-4  -3  -2  -1  0                    6   7   9        13  14                    19  20        22

LAB: voie intra-gastrique
TNBS 1mg/souris : voie intra-rectale
TNBS 2 mg/souris : voie intra-rectale
Sacrifice: scores macroscopique et histologique

Figure 1 : Protocole d'administration des souches probiotiques

**Temps (jours)**

-5  -4  -3  -2 -1  0            5     7          12    14    16

LAB: voie intra-gastrique
TNBS 2 mg/souris : voie intra-rectale
Sacrifice: scores macroscopique et histologiques

Figure 3 : Protocole d'administration des souches probiotiques

**Temps (jours)**

-5  -4  -3  -2  -1  0        2

LAB: voie intra-gastrique
TNBS: 100 mg/kg, voie intra-rectale
Sacrifice: scores macroscopique et histologique

Figure 5 : Protocole d'administration des souches probiotiques

Figure 2 : score de Wallace et d'Ameho, et évolution du poids

Figure 4 : scores de Wallace et Ameho

Figure 6 : scores de Wallace

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **CAMOGLIO et al.** *Eur J Immunol,* 2000

- **ROSDENBERG et al.** *FEMS Microbiol. Letters,* 1980, vol. 9, 29-33